# EUROPEAN PATENT SPECIFICATION

(11) **EP 1 743 665 B1**
(45) Date of publication and mention of the grant of the patent: **11.03.2015**
(21) Application number: 05015413.7
(22) Date of filing: 15.07.2005
(51) Int. Cl.: A61M 5/142

(54) **Neurotransmitter stimulation of neurons with feedback from sensors**
Neuronenstimulation mittels Neurotransmitter unter Rückkoppelung von Sensoren
Stimulation de neurones à l'aide de neurotransmetteurs avec rétroaction des capteurs

(43) Date of publication of application: 17.01.2007
(73) Proprietor: IMEC, 3001 Leuven (BE)
(72) Inventor: Bartic, Carmen, B-3012 Wilsele (BE); Mernier, Guillaume, B-3052 Blanden (BE)
(74) Representative: Bird, William Edward

(56) References cited:
- AT-B- 407 708
- US-A- 4 750 499
- US-A1- 2004 224 002
- US-A1- 2004 230 270

## Description

The present invention relates to a neurotransmitter release system for on-chip stimulation of neurons by means of neurotransmitters and to a method for manufacturing such a system. The system according to the invention comprises an actuated system for chemical stimulation (ASyCS) of neurons and a feedback system for controlling the actuation. The neurotransmitter release system may be used in the field of biomedical devices, including implants, biosensors and actuators.

### Background of the invention

The human brain comprises lots of, i.e. billions of, neurons, which are mutually interconnected. These neurons get information from sensory nerves and provide a motor feedback to the muscles. Neurons can be stimulated in two ways, i.e. electrically and chemically. Neurons are living cells which comprise a cell body and different extensions and which are delimited by a membrane. Differences in ion concentrations inside and outside the neurons cause a voltage across this membrane. The membrane is impermeable to ions but comprises proteins that can act as ion channels that can open and close, enabling ions to flow through the membrane. Opening and closing of the first type may be controlled by applying a voltage and thus is electrically stimulated while the second type may be activated by binding of a specific molecule on it, and thus is chemically stimulated.

When a neuron is stimulated, an electrical signal, which may also be called action potential, is created across the membrane. This signal is transported along the longest extension, called the axon, of the neuron towards another neuron. The two neurons are not physically connected to each other: at the end of the axon, a free space, which called the synaptic cleft, separates the membrane of the stimulated neuron from the next neuron. For transferring the information to the next neuron, the first neuron has to transform the electrical signal into a chemical signal by the release of specific chemicals called neurotransmitters. These molecules diffuse in the synaptic cleft and bind on specific receptors, i.e. proteins, on the second neuron. The binding of a single neurotransmitter molecule can open an ion channel in the membrane of the second neuron and allows thousands of ions to flow through it, rebuilding an electrical signal across the membrane of the second neuron. This electrical signal is then transported again along the axon of the second neuron and stimulates the next one, i.e. a third neuron.

To study the behaviour of neurons, it has been tried to couple them to microelectronic devices in order to stimulate the neurons and record the created action potentials. Systems based on bi-directional communication find applications in the field of implantable devices, biosensors and model systems for bio-medical research.

Up till now, research has mostly been focused on electrical stimulation of neurons by means of extra-cellular electrodes, i.e. the application of voltage pulses of several volts causes a voltage drop across the neuron membrane, which should be high enough to create an action potential. For example, the voltage drop across the neuron membrane may be several tens of millivolts ['P. Fromherz, "Neuroelectronic Interfacing: Semiconductor Chips with Ion Channels, Nerve Cells, and Brain", Nanoelectronics and Information technology, 781-810 (2003)' and 'A. Cohen et al.; 'Depletion type floating gate p-channel MOS transistor for recording action potentials generated by cultured neurons", Biosensors and Bioelectronics, vol.19, 1703-1709 (2004)']. This technique, however, is not efficient because a high voltage has to be applied, and its efficiency and reproducibility depend on the quality of the coupling layer between the neuron and the chip.

Therefore, a new approach has been proposed which uses neurotransmitter stimulation in order to increase the efficiency/reproducibility of on-chip cell stimulation. In this approach, neurotransmitter molecules are released on a chip to chemically stimulate the neurons. Neurotransmitters have already been used to stimulate retina cells on chip [M.C. Peterman et al., "Localized chemical release from an artificial synapse chip", PNAS, vol. 101, no.27, 9951-9954 (2004)], but the system described stimulates several cells within a radius of 25 µm around the release site and not individual cells. As this system is not coupled to biosensors, it is not possible to create feedback. Furthermore, in the system described in the above reference, the stimulation of neurons is only being checked by fluorescent means. This is a visual check of the stimulation, which implies that the release of neurotransmitters has to be stopped or controlled manually.

For studying neurons in vivo, brain probes may be used. Known brain probes focus on electrical stimulation, e.g. the brain probe of MedTronics, described in US 2002/0022872. Neurotransmitter delivery has also been performed using a brain probe comprising microfluidic chemical delivery as well as transistors for signal recording [R. Rathnasingham, D.R. Kipke, S.C. Bledsoe Jr en J.D. McLaren, "Characterization of Implantable Microfabricated Fluid Delivery Devices", IEEE Transactions on Biomedical Engineering, vol.51, no.1 (2004)]. This probe is externally-driven by a microsyringe and not integrated on a chip. However, the above-described system has to be actuated externally. The probe is injected in a patient's brain and the delivery of the chemicals occurs by means of a micro-syringe situated outside the patient's body. This means that the control is thus external and not integrated on the system that is implanted.

A system according to the preamble of claim 1 is known from US 2004 224 002.

### Summary of the invention

It is an object of the present invention to provide an on-chip neurotransmitter release system for performing improved neuron stimulation. A potential advantage of the system according to the invention is that it can stimulate individual neurons in a more efficient way than prior art systems and is coupled to a feedback system for providing feedback from the neurons to the neurotransmitter release system. Through this, the system is more stable and therefore suitable for bio-medical applications.

The above objective is accomplished by a method and device according to the present invention.

In a first aspect of the invention, a system for performing neurotransmitter stimulation of neurons is provided. The system comprises an actuated system for the chemical stimulation (or ASyCS) of neurons and a feedback system. According to the first aspect of the invention, the actuated system for the chemical stimulation (or ASyCS) of neurons comprises:
- at least one synthetic reservoir with at least one aperture, the synthetic reservoir comprising neurotransmitter molecules, and
- an actuation means for controlling release of neurotransmitter molecules through the at least one aperture of the at least one synthetic reservoir,
wherein the actuation means is controlled by the feedback system.

The system according to the first aspect of the invention provides chemical stimulation to neurons. The advantage of chemical stimulation over conventional devices using electrical stimulation is a much smaller power consumption. This enhances the lifetime of the power supply, and thus the amount of time between two surgical operations. Furthermore, the system according to the first aspect of the invention is an on-chip system, wherein actuation does not have to occur externally.

The system according to the invention can be used for single neuron stimulation, is efficient, reproducible and requires a lower power supply than prior art systems.

According to embodiments of the invention, the feedback system may comprise at least one sensor, for example, at least one biosensor. The feedback system may be used for controlling the actuation means such that, whenever required, release of neurotransmitters may be stopped or restarted depending on the control signal coming from the feedback system.

According to embodiments of the first aspect of the invention, the actuation means may be an electrically driven actuation means. In one embodiment, the electrically driven actuation means may comprise at least a first and a second electrode. The at least one synthetic reservoir may be formed in a substrate and the first electrode may be positioned at a bottom surface of the substrate and the second electrode may be positioned at a top surface of the substrate.

By applying an electrical signal, e.g. a voltage, between the first electrode and the second electrode the neurotransmitter molecules are transferred through the aperture in the reservoir towards an external environment.

According to other embodiments of the invention, the actuation means may be a pressure-based actuation means. In one embodiment, the pressure-based actuation means may comprise a membrane. The membrane may be formed of an electrically actuatable layer sandwiched between two electrodes. In specific embodiments, the electrically actuatable layer may be a piezoelectric layer and may for example be a ZnO film, a PZT film or an AIN film.

Alternatively, the actuation means may comprise one single electrode and a polymer layer on top of it. The polymer layer may, for example, be polypyrole.

In the case where the membrane is formed of a piezoelectric layer sandwiched between two electrodes, an electrical signal, e.g. voltage, may be applied between the first and second electrodes and thus across the piezoelectric membrane. Because of that, the membrane will bend and in that way cause an overpressure within the synthetic reservoir. Due to the overpressure, neurotransmitter molecules are released through the aperture, out of the synthetic reservoir towards an external environment.

According to embodiments of the invention, the system may comprise a first and a second substrate. The pressure-based actuation means may be positioned in between the first and second substrate.

According to embodiments of the invention, the actuation means may be dimensioned such that the system can be used for single neuron stimulation.

According to embodiments of the first aspect of the invention, the system may comprise a plurality of synthetic reservoirs, for example an array of synthetic reservoirs, wherein each reservoir has an aperture. An advantage of this implementation is that the system can be used for single neuron stimulation.

In other embodiments of the first aspect of the invention, the system may comprise a plurality of reservoirs, for example an array of synthetic reservoirs, wherein at least one synthetic reservoir comprises more than one aperture.

In a second aspect of the invention, a method is provided for the manufacturing of a system for performing neurotransmitter stimulation of neurons. The method comprises:
- providing at least one synthetic reservoir (2) for containing neurotransmitter molecules (3),
- providing said at least one synthetic reservoir (2) with at least one aperture (9),
- providing actuation means for controlling release of neurotransmitter molecules (3) through said at least one aperture (9) of said at least one synthetic reservoir (2), and
- providing feedback means for controlling the actuation means.

According to embodiments of the invention, the feedback system may comprise at least one sensor, for example, at least one biosensor.

According to embodiments of the second aspect of the invention, providing at least one synthetic reservoir may comprise etching a substrate from a first surface toward a second surface of the substrate.

According to embodiments of the invention, providing actuation means may comprise providing electrically driven actuation means. In one embodiment, providing actuation means may be performed by providing a first electrode on a first surface of the substrate and providing a second electrode on a second surface of the substrate. In this case, for the release of neurotransmitters from the synthetic reservoir, an electrical signal, e.g. voltage, may be applied between the first and second electrode.

In other embodiments according to the second aspect of the invention, providing actuation means may comprise providing pressure-based actuation means. In one embodiment, providing actuation means may be performed by providing an electrically actuatable membrane. In embodiments of the invention, this may be performed by providing a film, for example a piezoelectric film, sandwiched in between two electrodes. For the release of neurotransmitters out of the synthetic reservoir, an electrical signal, e.g. voltage, may be applied between the first and second electrodes and thus across the piezoelectric membrane. Because of that, the membrane will bend and in that way cause an overpressure within the synthetic reservoir. Due to the overpressure, neurotransmitter molecules are released through the aperture out of the synthetic reservoir towards an external environment.

In a third aspect of the invention, a method is provided for determining a control signal for controlling actuation means of a system according to the present invention. The method comprises:
- detecting neuron activity in the form of neuronal signals,
- comparing said neuronal signals with a pre-determined threshold voltage, and
- from this comparison, determining the control signal for the actuation means.

The present invention furthermore provides a computer program product which when executed on a processing device executes the method for determining an actuation signal for controlling actuation means of a system according to the invention and a machine readable data storage device storing the computer program product according to the invention.

Particular and preferred aspects of the invention are set out in the accompanying independent and dependent claims. Features from the dependent claims may be combined with features of the independent claims and with features of other dependent claims as appropriate and not merely as explicitly set out in the claims.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

Fig. 1 illustrates an ASyCS system according to an embodiment of the invention.
Fig. 2 illustrates possible implementations for the second electrode in the system of Fig. 1.
Fig. 3 illustrates an ASyCS system according to an embodiment of the invention.
Fig. 4 schematically illustrates possible applications for the system according to the present invention.
Fig. 5 illustrates a brain probe implanted in the brain.

In the different figures, the same reference signs refer to the same or analogous elements.

### Description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

In the present description, the terms "neurotransmitters" and "neurotransmitter molecules" are both used, and both have the same meaning. By neurotransmitter is meant a chemical substance that naturally occurs in a brain of a living organism and that is responsible for communication among nerve cells.

The present invention provides a system for performing neuron stimulation, which, hereinafter, will be referred to as Actuated System for the Chemical Stimulation or ASyCS of neurons. In the further description this will be referred to as the ASyCS system. According to the invention, the ASyCS system is coupled to a feedback system for providing feedback. The feedback may then be used to control the release of neurotransmitters from the ASyCS system, i.e. to stop or restart the release of the neurotransmitters. In other words, the feedback allows control of the actuation of the ASyCS system.

The ASyCS system according to the invention is an on-chip system, this means that it is fabricated and integrated at least partly in a substrate. This is different from other known prior art systems used to deliver e.g. chemicals to e.g. a body of a patient, such as e.g. pipettes or injection needles.

The ASyCS system according to the present invention is able to locally deliver neurotransmitter molecules in a controlled way. It may be used for, for example, in vitro stimulation of individual neurons and in neuro-physiological research. The ASyCS system may also be used in vivo, for example in brain-controlled prostheses and implants. Prostheses can replace damaged parts of the human body, such as e.g. the limbs and the retina. Implants can be used to threat neurological disorders, such as Parkinson or epilepsy.

As the ASyCS system according to the invention is working with neurons, this implies several limitations such as, for example, on temperature and voltage. The neurons have to be kept at physiological temperature and a difference of several degrees in temperature could affect the neuronal activity. Another important limitation is the voltage that can be used, as this can affect the ASyCS system in different ways. First of all, the application of several volts would change the way of stimulation from chemical stimulation (with neurotransmitters) to electrical stimulation. For example, depending on the geometry of the system used, on the form of the applied signal and on a coupling layer between the neuron and the chip, electrical stimulation can occur starting from 3 to 4 V. Another limitation may come from the aqueous environment in which the neurons live in the brain. Immersed electrodes undergo electrochemical reactions under the application of a voltage depending on the metal used, but which typically may be in the range of 1 V. This reaction will dissolve the electrode and create compounds that can be lethal for the neurons.

In order to stimulate one neuron, a certain amount of neurotransmitter molecules or neurotransmitters has to be released. This amount depends on the type of neurons to be stimulated and on the geometry of the ASyCS system. Therefore, according to the invention, the ASyCS system comprises a feedback system which is needed to stop the release of neurotransmitter molecules after neuron stimulation and to restart the release of neurotransmitter molecules whenever required. The feedback system may, for example, comprise at least one sensor, e.g. at least one biosensor.
The ASyCS system according to the present invention comprises at least one reservoir which is filled with neurotransmitter molecules. Furthermore, the ASyCS system according to the invention comprises access channels or apertures between the reservoir and the environment containing neurons, e.g. the brain, and an actuation system to control the delivery of the neurotransmitters. This actuation system may be implemented by different means, e.g. a pressure-based actuation means or an electrically-based actuation means (see further).

In a first embodiment of the invention, the ASyCS system is an electrically-driven system. Electrically-driven systems use electro-kinetics to control the release of the neurotransmitter molecules. In that case, the neurotransmitter molecules move by the application of an external voltage and due to electrostatic interaction.

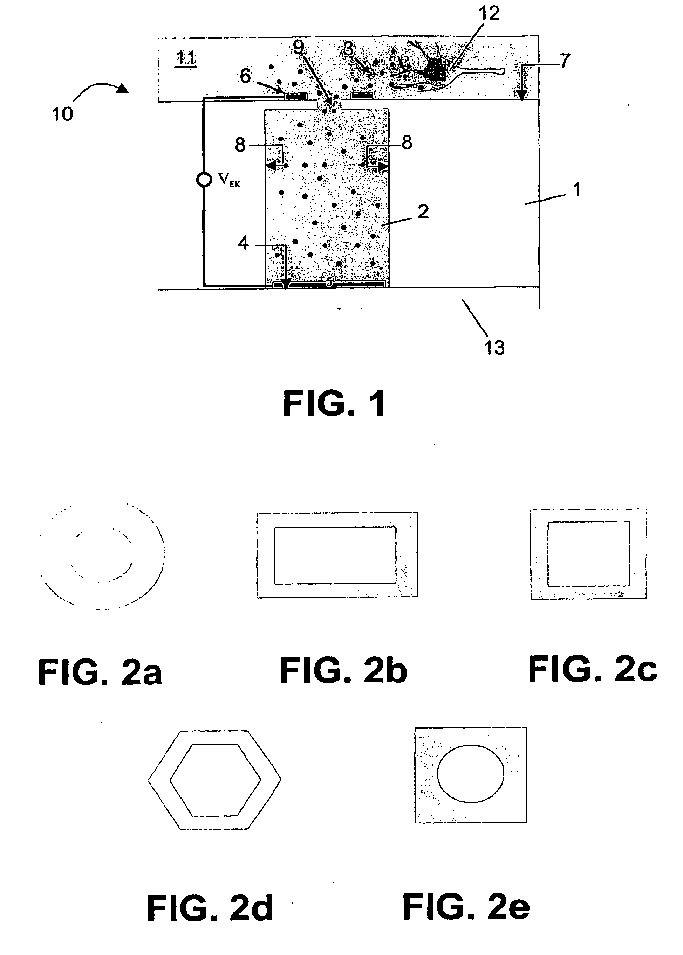

Fig. 1 shows a cross-section of a possible implementation of such system according to the first embodiment of the invention. Hereinafter, the example given in this figure will be described. It has, however, to be understood that this is only for the ease of explanation and this example is not limiting the invention. Other implementations of electrically-driven systems may be possible.

The ASyCS system 10 according to the first embodiment of the invention comprises a substrate 1. The substrate 1 may comprise a semiconductor material, (preferably Si but also other semiconductor materials may be used), glass coated with a biocompatible material, polymers (e.g. polyurethanes or polyimides) or biocompatible silicones. The substrate 1 may have a thickness of between 100 µm and 2 mm, preferably between 100 µm and 0,5 mm and more preferably between 100 µm and 200 µm. The substrate 1 comprises a reservoir 2 which is filled with neurotransmitters 3. Examples of neurotransmitters 3 which may be used according to the invention are summarised in table 1. It has however to be understood that this is only by way of illustration and thus is not limiting the invention. Other neurotransmitters 3 may be used as well.

**Table 1: Examples of neurotransmitters that can be used with the present invention.**

| | | | | | |
|---|---|---|---|---|---|
| 1. | L-GLUTAMATE | 8. | KRYPTOPYRROLE ACETYLCHOLINE | 15. | TRYPTOPHAN GAMMA-AMINOBUTYLIC ACID (GABA) |
| 2. | D-GLUTAMATE | | | | |
| 3. | ACETYLCHOLINE CHLOR | 9. | L-DOPA | | |
| | | 10. | L-GLUTAMIC ACID | 16. | ORTHOMETHYL SEROTONIN |
| 4. | DOPAMINE | 11. | L-GLUTAMINE | 17. | PHENYLETHYLAMINE |
| 5. | VITAMIN B-6 | 12. | MALVIN | 18. | SEROTONIN |
| 6. | HISTAMINE | 13. | NITRIC OXIDE | 19. | TAURINE |
| 7. | HISTIDINE | 14. | NOREPINEPHRINE | | |

Furthermore, the ASyCS system 10 according to the first embodiment of the invention comprises a first electrode 5 and a second electrode 6. The first electrode 5 may be positioned on the bottom 4 of the substrate 1, at the position of the reservoir 2, and the second electrode 6 may positioned on the top surface 7 of the substrate 1. However, in other embodiments, the first electrode 5 and the second electrode 6 may be positioned in another way. For example, the first electrode 5 may be at least partially positioned on the inner side walls 8 of the reservoir 2. In other embodiments according to the invention, the ASyCS system 10 may comprise two first electrodes 5, either both positioned on the bottom 4 of the reservoir 2 and/or each of the two first electrodes 5 may be at least partially positioned at inner side walls 8 of the reservoir 2. In the example given in Fig. 1 the second electrode 6 may have an open circular shape (see Fig. 2a). However, the second electrode 6 may also have an open rectangular shape (see Fig. 2b), an open square shape (see Fig. 2c) or an open polygonal shape (see Fig. 2d), or may have any possible suitable shape, such as a square shape with a circular hole in it (see Fig. 2e). The second electrode 6 positioned as illustrated in Fig. 1, may have a shape such that it allows access to the reservoir 2. However, in embodiments according to the invention, the second electrode 6 may be positioned nearby an aperture 9, suitable for the release of neurotransmitters 3 out of the reservoir 2 into an external environment 11. In that case, the second electrode 6 may have any suitable shape. In still further embodiments, the second electrode 6 may comprise more than one electrode. The position of these electrodes may influence the direction to which neurotransmitter molecules 3 are released.

The application of an electrical signal, e.g. a voltage, between the first electrode 5 and the second electrode 6 transfers the neurotransmitter molecules 3, for example glutamate molecules, through the aperture 9 in the reservoir 2 towards an external environment 11. The applied voltage may be between 0.1 V and 3 V, preferably between 0.1 V and 2 V, more preferably between 0.5 V and 1.5 V and most preferably between 0.5 V and 1 V. The neurotransmitter molecules 3 then bind on specific receptors of the neurons 12 which are e.g. present in the brain in which the system 10 is implanted.

The size of the system 10 depends on the application. The reservoir 2 may have sizes in the order of several mm.

According to embodiments of the invention, the substrate 1 may comprise one big reservoir 2 having different apertures 9 for providing neurotransmitters 3 to different sites or may, in other embodiments, comprise a plurality of individual reservoirs 2, e.g. at least two individual reservoirs 2, each comprising neurotransmitters and each having one or more apertures 9. In one substrate 1, there can be provided thousands or even millions of reservoirs 2. In that case, the reservoirs 2 may, for example, be ordered in dense arrays in order to stimulate single neurons 12. The size of the reservoir 2 depends on whether there is only one reservoir 2 feeding a plurality of apertures 9 or whether there is a reservoir 2 for each aperture 9. It has to be understood that in the first case the reservoir 2 should preferably be larger than in the latter case. In the latter case, the reservoirs 2 can be quite large, i.e. in the range of hundreds of micrometers up to even 10 mm, to allow many different stimulations, or smaller, i.e. in the range of tens of micrometers, to get a better spatial resolution. Hence, the choice of the size of the reservoirs 2, which may be between 10 µm and 10 mm, depends on the application. The size of the apertures 9 can also vary from several microns down to several nanometers, depending on the application.

The system 1 according to the first embodiment of the invention furthermore comprises a sealing layer 13 for closing the reservoir 2 present in the substrate 1 (see further).

Hereinafter, a method for the fabrication of the ASyCS system according to the first embodiment of the invention will be described.

For the manufacturing of the system 10 as illustrated in Fig. 1, in a first step a substrate 1 is provided. The second electrode 6 may be provided on the top surface 7 of the substrate 1 by means of any suitable technique known by a person skilled in the art, such as e.g. by means of a lift-off technique. In a next step, an aperture 9 is formed from the top surface 7 of the substrate 1 toward the bottom surface 4 of the substrate 1. The aperture 9 may have a width of between 1 nm and 20 µm and may have a depth of between 10 nm if the apertures 9 are formed in e.g. a membrane and tens of µm if longer micro/nanochannels are connecting the reservoir 2 and the external environment.

Formation of the aperture 9 may, for example, be done by etching. Etching may be performed by any suitable technique known by persons skilled in the art, e.g. (Deep) Reactive Ion Etching (D)RIE or e-beam etching, depending on the size and shape of the aperture 9 that is required for particular applications.

In a next step, a reservoir 2 is made, e.g. etched, preferably from the bottom surface 4 of the substrate 1 toward the top surface 7 of the substrate 1. Again, etching may be performed by any suitable etching technique known by persons skilled in the art, e.g. by DRIE. The reservoir 2 may have a depth that is equal to the thickness of the substrate 1 minus the depth of the aperture 9 that is formed in the substrate 1. The aperture 9 provides access between the reservoir 2 and external environment 11.

Next, a sealing layer 13 for sealing, i.e. closing off, the reservoir 2 may be attached, e.g. sealed or glued, onto the bottom surface 4 of the substrate 1. This sealing layer 13 may, for example, comprise flexible and easy to process polymers like e.g. PolyDiMethylSilane (PDMS), polyimide, polyurethane. The material for the sealing layer 13 should be impermeable for the neurotransmitter solution, able to bond to the material of the substrate 1 in which the reservoir 2 is formed and should preferably be flexible. The thickness of the sealing layer 13 is not critical and thus the sealing layer 13 may have any suitable thickness.

Before the sealing layer 13 is attached to the bottom surface 4 of the substrate 1, an electrode, forming the first electrode 5, is deposited onto that side of the sealing layer 13 which will form the inner bottom side of the reservoir 2. This may be done by any suitable technique known by persons skilled in the art. The first electrode 5 is aligned with the reservoir 2. Alignment of the first electrode 5 with the reservoir 2 and attachment of the sealing layer 13 to the substrate 1 may for example be obtained by using flip-chip bonding technology. The first electrode 5 and the second electrode 6 may be formed of an electrically conductive material, such as e.g. a metal or any other suitable electrically conductive material. The conductive material of the first and second electrodes 5, 6 has to be cytophilic and easy to process. It may, for example, be platinum or gold. For enhancing the adhesion of the electrodes 5, 6 to the substrate 1, an adhesion layer, which may be made of e.g. titanium, chromium or tungsten, may be first deposited onto the substrate 1. This may be done by conventional techniques known by a person skilled in the art.

It has to be noted that in one substrate 1, a plurality individual ASyCS systems 10 may be positioned. The number of ASyCS systems 10 depends on the size of the substrate 1 in which the ASyCS systems 10 are formed. Furthermore, it should be taken into account that the individual ASyCS systems 10 are positioned no closer to each other than the size of the neurons 12 to be stimulated. The distance between neighbouring ASyCS systems 10 may typically be about 10 µm.

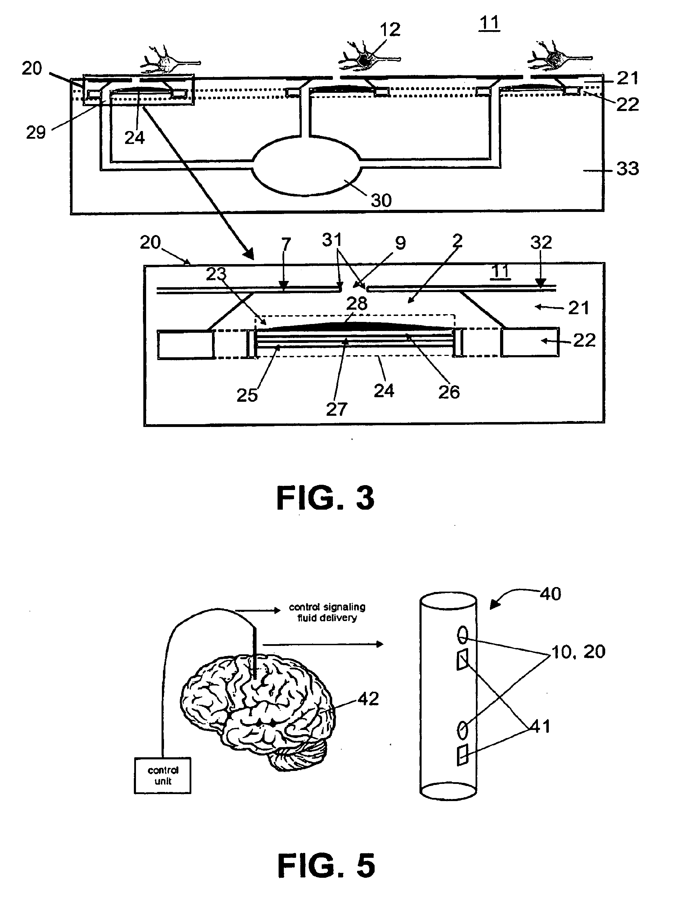

Another way to actuate the ASyCS system is pressure-based. In a second embodiment, pressure-based systems will be described. Fig. 3 illustrates a possible implementation of such a system, using a piezoelectric phenomenon. It has to be understood that this is only by means of explanation and that this example is not limiting the invention. Other implementations of pressure-based systems are also covered by the present invention.

The pressure-based system 20 according to the second embodiment of the invention comprises a first substrate 21 and a second substrate 22. The second substrate 22 is meant for deposition of an actuatable membrane thereon (see further) and for closing the reservoir 2. When the reservoir 2 is formed by etching the first substrate 21, this results in a substrate 1 with an open side. Therefore, the second substrate 22 with the actuatable membrane on it is used to close of the open side of the first substrate 21 (see further).

The first and second substrates 21, 22 may comprise a semiconductor material, (preferably Si but also other semiconductor materials may be used), glass coated with a biocompatible material, polymers (e.g. polyurethanes or polyimides) or biocompatible silicones. The first substrate 21 may have a thickness of between 1 µm and 100 µm. The thickness of the first substrate 21 determines the height of the reservoir 2 and should preferably not be too large in order to get a higher difference in pressure within the reservoir 2 during actuation.

The first substrate 21 comprises a chamber or reservoir 2. In this reservoir 2 neurotransmitter molecules (not shown in the figure) are stored. At the top surface of the reservoir 2, an aperture 9 is provided, for releasing neurotransmitters to the external environment. At the bottom surface 23 of the first substrate 21, more particularly at the level of the reservoir 2, an actuatable membrane 24 is provided. The actuatable membrane 24 may be a piezoelectric membrane 24, which has a shape suitable for closing off the reservoir 2, e.g. a circular shape. The actuatable membrane 24 in that case comprises a thin film of piezoelectric material sandwiched between two electrodes. Alternatively, the actuatable membrane 24 may be formed of one single electrode and a polymer layer on top of it. The polymer layer may, for example, comprise polypyrole.

In the example given in Fig. 3, the actuatable membrane is a piezoelectric membrane 24, formed of two electrodes 25, 26 and a piezoelectric material 27 sandwiched in between the electrodes. A piezoelectric material 27 bends when a voltage is applied across it. To apply this voltage, at least two electrodes 25, 26 may be required. For forming the piezoelectric membrane 24 as illustrated in Fig. 3, a first electrode 25, the piezo-material 27 and a second electrode 26 may be deposited on top of each other. The black oval indicated by reference number 28 in Fig. 3 schematically illustrates a displacement of the piezoelectric membrane 24 during actuation.

When an electrical signal, e.g. voltage, is applied between the first and second electrodes 25, 26 and thus across the piezoelectric membrane 24, the membrane 24 will bend and in that way cause an overpressure within the reservoir 2. Due to the overpressure, neurotransmitter molecules are released through an aperture 9 out of the reservoir 2 towards the external environment 11. The voltage to be applied may depend on different parameters, i.e. the piezoelectric material 27 used, the size of the membrane 24, the displacement that is required, ... The voltage may be between 0.1 and 10 V. The voltage may be applied at the first or bottom electrode 25 while the second or top electrode 26 is grounded, so that the actuation potential is screened by the membrane 24.

When the voltage ceases to be applied, the membrane 24 returns to its equilibrium position and the reservoir 2 is filled through an inlet 29, which is connected to a central reservoir 30 as shown in the upper part of Fig. 3. In case the ASyCS system 20 according to the second embodiment of the invention comprises a plurality of reservoirs 2, as illustrated in the upper part of Fig. 3, the central reservoir 30 may feed all the individual reservoirs 2. Alternatively, a plurality of central reservoirs 30 may be provided to feed the individual reservoirs 2.

In embodiments according to the present invention, a thin film, e.g. piezoelectric film such as a ZnO film, a PZT film or an AIN film, may be used instead of a large piezoelectric membrane for forming the actuatable membrane for closing off the reservoir 2. This may reduce the voltage required for actuation of the closing membrane 24 from hundreds of volts down to a few volts. The thin film may be actuated at its resonance frequency to get a higher expulsion force. To avoid undesired effects due to the voltage used for actuation, the top electrode 26, which is in contact with the neurotransmitter molecules in the reservoir 2, may be grounded and the actuation signal may be applied at the second or bottom electrode 27 below the thin film.

To prevent diffusion of the neurotransmitter molecules out of the reservoir 2 when no voltage is applied to the ASyCS system 20, i.e. when the ASyCS system is in the OFF state, e.g. surface chemistry can be used to make the side walls 31 of the aperture 9 hydrophobic, thereby forming a diffusion barrier.

The size of this system 20 according to the second embodiment of the invention is more critical than for the system 10 of the first embodiment of the invention. The relative excess of pressure in the reservoir 2 depends on the ratio between the volume of neurotransmitter molecules displaced by the piezoelectric membrane 24 and the total volume of neurotransmitter molecules in the reservoir 2. This ratio has to be as high as possible to get efficient release of neurotransmitter molecules through the aperture 9. In case the actuatable membrane 24 has a circular shape, its diameter may range from tens of microns to hundreds of microns. The size of the aperture 9 may be in the range of several microns, and the height of the reservoir 2 may be in the range of tens of microns.

Hereinafter, a method for the fabrication of the pressure-based system 20 according to the second embodiment of the invention and illustrated in Fig. 3 will be described.

For the fabrication of this system, different substrates are needed. A first substrate 21 is provided. The first substrate 21 may comprise a semiconductor material, (preferably Si but also other semiconductor materials may be used), glass coated with a biocompatible material, polymers (e.g. polyurethanes or polyimides) or biocompatible silicones. In other embodiments according to the invention, a silicon on insulator (SOI) may also be applied for the first substrate 21. The first substrate 21 may furthermore comprise a capping layer 32, which may preferably be a silicon nitride layer, on its top surface 7.

The capping layer 32, e.g. silicon nitride layer, may, for example, have a thickness of between 100 and 500 nm. An aperture 9 may be provided by etching the capping layer 32, e.g. silicon nitride layer, by means of suitable etching techniques known by persons skilled in the art, e.g. selective etching of the capping layer 32, e.g. silicon nitride layer, with respect to the first substrate 21 in which the first substrate 21 acts as a stopping layer.

In a next step, the reservoir 2 may be formed into the first substrate 21 by any suitable method, e.g. by means of etching the first substrate 21 from its bottom surface 23 toward the top surface 7, using, for example, anisotropic wet etching. The capping layer 32, e.g. silicon nitride layer, at its top surface may then act as a stopping layer and etching may be continued so long that part of the capping layer 32, e.g. silicon nitride layer, becomes a free-standing layer. Typically, the solidity of silicon nitride is sufficient to withstand microfabrication. However, in cases where a very robust layer is required for withstanding the vibration of the actuatable membrane, e.g. piezoelectric membrane, the silicon nitride layer 32 can be reinforced by a reinforcing layer, such as e.g. a silicon oxide layer (not shown in the figure). The combination of these two materials, the silicon oxide layer having mechanical properties different from the ones of the silicon nitride layer 32, i.e. tensile stress for nitride and compressive stress for oxide, makes it very strong thanks to stress compensation. In case a silicon on insulator (SOI) substrate is used, the reservoir 2 may also be etched from the bottom surface 23 toward the top surface 7 of the first substrate 1, 2 whereby the silicon layer on top of the insulator will reinforce the solidity of the capping layer 32 separating the reservoir 2 and the external environment 11. The first substrate 21 may then be thinned down to 1 to 100 µm to reduce the height of the reservoir 2.

In Fig. 3 the reservoir 2 has the shape of a frustum. This is because in this case wet etching with a KOH solution has been used for forming the reservoir 2 in the first substrate 21. However, it has to be understood that also reservoirs 2 having other shapes are included in this invention. For example, by using dry etching also cylindrical or square reservoirs 2 can be formed.

A second substrate 22 is then provided. The second substrate 22 may comprise a semiconductor material (preferably Si but also other semiconductor materials such as e.g. GaAs may be used), glass coated with a biocompatible material, polymers (e.g. polyurethanes or polyimides) or biocompatible silicones and may first be etched by, for example, RIE from its top surface to create a small cavity. An actuatable membrane 24, which may be a piezoelectric layer or a thin film membrane 27 surrounded by electrodes 25, 26, is then provided in this cavity. This may be done by subsequently depositing a first or bottom electrode 25, a piezoelectric material 27 and a second or top electrode 26. This may be done by conventional techniques known by persons skilled in the art, such as e.g. evaporation or sputtering. The method used for deposition of the first and second electrode 25, 26 depends on the type of conductive material, e.g. metal, used for making up the electrodes 25, 26.

In a next step the second substrate 22 is etched from its bottom surface toward the top surface using a suitable etching method such as e.g. DRIE, in order to make the actuatable membrane 24 free-standing. Electrodes 25, 26 are provided at either side of the membrane 24, and may, for example, comprise platinum or aluminum. When the membrane 24 is a piezoelectric membrane, different piezoelectric materials can be used, such as, for example, PZT, ZnO and AIN. In a next step, inlets 4 may furthermore be provided, e.g. may also be etched using DRIE, in the second substrate 22 for access to a central reservoir 30.

An additional substrate 33 may then comprise the central reservoir 30. This additional substrate 33 may be made of polymers like PDMS, polyurethane or polyimide. When the different substrates, i.e. first substrate 21, the second substrate 22 and the additional substrate 33, are bonded, the fabrication of the pressure-based ASyCS system is complete. Bonding of the second substrate 22 to the additional substrate 33 may be performed by e.g. gluing.

Hereinafter, possible applications of the ASyCS system 10, 20 according to the invention will be described.

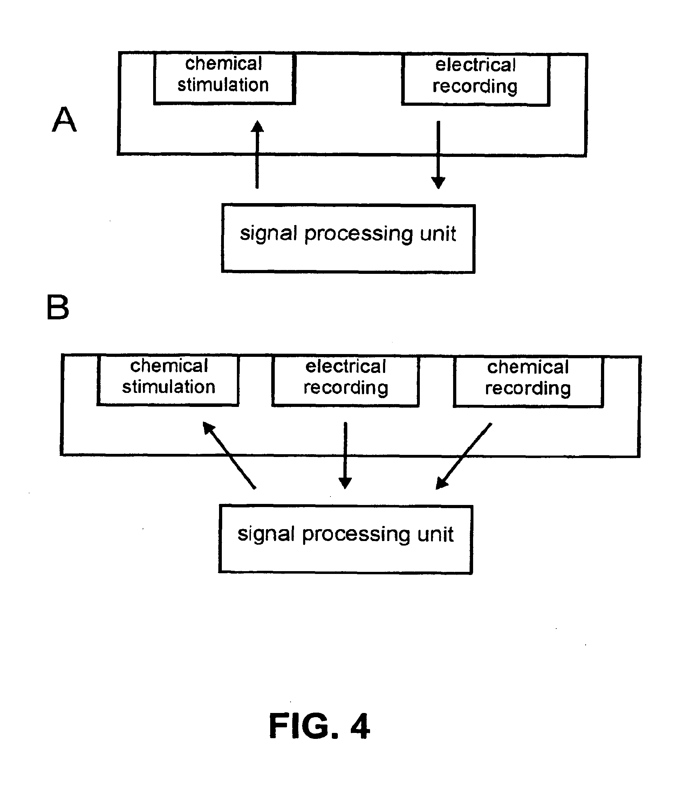

In Fig. 4 an overview is given of possible ways the ASyCS system 10, 20 according to the invention can be applied. According to embodiments of the invention, the ASyCS system 10, 20 can be used in combination with electrical sensors (Fig. 4A). These sensors can detect neuronal action potentials and provide feedback to the ASyCS system 10, 20. When the neurotransmitter release occurs, the neurotransmitter molecules 3 diffuse towards the neurons 12 and stimulate them. The amount of neurotransmitter molecules 3 needed to stimulate is difficult to predict. It should be in the range of thousands to millions of molecules, giving around one picoliter, depending on the concentration. It depends tremendously on the type of neurotransmitters 3 used and on the geometry of the configuration. That is why a feedback system is provided to control the release in order to release the right amount of neurotransmitter molecules 3. When the closest neuron 12 is stimulated, it triggers an electrical signal that can be detected by the sensor. The sensor can then, by the way of a central processing unit, send a signal to the ASyCS system 10, 20 to stop the delivery of the neurotransmitter 3. This allows the invention to stimulate individual neurons 12, a feature which is required in implants and prostheses.

In the case of implants, such as implantable brain probes 40 as illustrated in Fig. 5, neurotransmitter stimulation can be used as treatment for neurological disorders e.g. Parkinson's disease and epilepsy. The probe 40 comprises an electrode comprising a plurality of ASyCS systems 10, 20 according to the invention and electrical sensors 41 as the feedback system, in that way providing stimulation of neurons 12 in the brain 42 and recording their activity. The advantage of chemical stimulation over conventional devices using electrical stimulation is a much smaller power consumption. This enhances the lifetime of the power supply, and thus the amount of time during two surgery operations.

In the case of prostheses, and especially retina prostheses, neurotransmitter stimulation is particularly relevant. A same neurotransmitter stimulation will cause different reactions for different neurons 12. This selectivity allows maintaining the natural pathways of stimulations occurring in natural retinas. In this case, a pattern of light is detected by an array of photodiodes. Each photodiode is coupled with an ASyCS system 10, 20 according to the invention and the pattern of light is transformed in a pattern of neurotransmitter release and thus of retina cell stimulation. Individual neuron stimulation enhances the resolution of the implant. Power consumption is an important problem for implantation, which can be solved by the use of neurotransmitter stimulation.

In a second possible application of the ASyCS system 10, 20 according to the invention, the ASyCS system 10, 20 can be used in combination with electrical and chemical sensors (Fig. 4B). In this case the neurons are cultured in vitro on a chip containing the different sensors. A chemical layer, for example a self-assembled monolayer or SAM is deposited on the chip to enhance the coupling between the neurons 12 and the sensors. Depending on the substrate used, the SAM may be formed of different molecules. For example, for gold substrates the SAM may be formed of thiols while for oxidised substrates the SAM may be formed of silane molecules. In other embodiments, a pattern of cytophobic and cytophilic materials may be provided. The system comprises a same feedback as the previous application method, but on top of that, chemical sensors can detect neurotransmitter release from the neurons 12. The sensors can thus monitor the electrical and chemical activity of the neurons in different environments. Furthermore, the chemical sensor can be used to detect the neurotransmitter delivered by the ASyCS system 10,20, and determine the amount of neurotransmitter 3 needed for neuron stimulation. The neuron-chip system becomes an in-vitro model of neuronal network, and can be used in neurophysiological research e.g. for Alzheimer's disease and in drug monitoring.

According to the invention, sensors, which are coupled to the AsyCS system 10, 20 are used for detecting neuronal signals. The neuronal signals so detected are then sent to a microcontroller that threats the information and then sends control signals to the actuation means of the release system or AsyCS system 10. In that way, actuation may be controlled and hence, release of neurotransmitters 3 may be controlled. Treatment of the information is performed by comparing the neuronal signals with a pre-determined threshold voltage, which may, for example, be several tens of mV. From this comparison the control signal for the actuation means may be determined.

The present invention furthermore includes a computer program product which provides, when executed on a computing device, the functionality of the method for determining a control signal for the actuation means using a system 10, 20 according to the present invention. Further, the present invention includes a data carrier such as a CD-ROM or a diskette which stores the computer program product in a machine readable form and which executes the method for determining a control signal for the actuation means using a system 10, 20 according to the present invention when executed on a computing device. Nowadays, such software is often offered on the Internet or a company Intranet for download, hence the present invention includes transmitting the computer program product according to the present invention over a local or wide area network. The computing device may include one of a microprocessor and an FPGA.

It is to be understood that although preferred embodiments, specific constructions and configurations, as well as materials, have been discussed herein for devices according to the present invention, various changes or modifications in form and detail may be made.

## Claims

1. A system for performing neurotransmitter stimulation of neurons (12), the system comprising an Actuated System for the Chemical Stimulation (10, 20) of neurons (12),
the actuated system for the chemical stimulation (10, 20) of neurons (12) comprising:
- at least one synthetic reservoir (2) with at least one aperture (9), the reservoir (2) comprising neurotransmitter molecules (3), and
- an actuation means for controlling release of neurotransmitter molecules (3) through the at least one aperture (9) of the at least one synthetic reservoir (2),
**characterized in that** said system for performing neurotransmitter stimulation of neurons further comprises a feedback system for providing feedback from the neurons (12) to the actuated system for the chemical stimulation (10, 20) of neurons; and **in that** the actuation means is controlled by said feedback system.

2. A system according to claim 1, wherein the feedback system comprises at least one sensor.

3. A system according to claim 1 or 2, wherein the actuation means is an electrically-driven actuation means.

4. A system according to claim 3, wherein the actuation means comprises at least a first electrode (5) and a second electrode (6).

5. A system according to claim 4, the at least one synthetic reservoir (2) being formed in a substrate (1), wherein the first electrode (5) is positioned at a bottom surface (4) of the substrate (1) and the second electrode (6) is positioned at a top surface (7) of the substrate (1).

6. A system according to claim 1 or 2, wherein the actuation means is a pressure-based actuation means.

7. A system according to claim 6, wherein the actuation means comprises a membrane (24).

8. A system according to claim 7, wherein the membrane (24) is formed of an electrically actuatable layer (27) sandwiched between two electrodes (25, 26).

9. A system according to claim 8, wherein the electrically actuatable layer (27) is a piezoelectric layer.

10. A system according to any of claims 6 to 9, the system comprising a first substrate (21) and a second substrate (22), wherein the actuation means is positioned in between the first substrate (21) and the second substrate (22).

11. A system according to any of the previous claims, wherein the system comprises an array of synthetic reservoirs (2), each synthetic reservoir (2) having an aperture (9).

12. A system according to any of claims 1 to 11, wherein the system comprises at least one synthetic reservoir (2) having more than one aperture (9).

13. A method for the manufacturing of a system for performing neurotransmitter stimulation of neurons (12), the method comprising:
- providing an actuated system for the chemical stimulation of neurons (12) comprising:
- providing at least one synthetic reservoir (2) for containing neurotransmitter molecules (3),
- providing said at least one synthetic reservoir (2) with at least one aperture (9), and
- providing actuation means for controlling release of neurotransmitter molecules (3) through said at least one aperture (9) of said at least one synthetic reservoir (2); and
- providing feedback means for providing feedback from the neurons (12) to the actuated system for the chemical stimulation (10, 20) of neurons, the feedback means being for controlling the actuation means.

14. A method according to claim 13, wherein providing feedback means comprising providing at least one sensor.

15. A method according to claim 13 or 14, wherein providing at least one synthetic reservoir (2) comprises etching a substrate (1, 21) from a first surface (11) toward a second surface (12) of the substrate (1, 21).

16. A method according to any of claims 13 to 15, wherein providing actuation means is performed by providing electrically-driven actuation means.

17. A method according to claim 16, wherein providing actuation means is performed by providing a first electrode (5) on a first surface (4) of the substrate (1) and providing a second electrode (6) on a second surface (7) of the substrate (1).

18. A method according to any of claims 13 to 15, wherein providing actuation means is performed by providing pressure-based actuation means.

19. A method according to claim 18, wherein providing actuation means is performed by providing an electrically actuatable membrane (24).

20. A method according to claim 19, wherein providing an electrically actuatable membrane (24) is performed by providing a film (27) sandwiched in between two electrodes (25, 26).

21. A method according to claim 20, wherein providing a film (27) is performed by providing a piezoelectric film.

## Patentansprüche

1. System zum Ausführen von Neurotransmitterstimulierung von Neuronen (12), wobei das System ein Betätigtes System zur Chemischen Stimulierung (Actuated System for the Chemical Stimulation) (10, 20) von Neuronen (12) umfasst,
wobei das betätigte System für die chemische Stimulierung (10, 20) von Neuronen (12) umfasst:
- zumindest einen synthetischen Behälter (2) mit zumindest einer Öffnung (9), wobei der Behälter (2) Neurotransmittermoleküle (3) umfasst, und
- ein Betätigungsmittel zum Steuern der Freisetzung von Neurotransmittermolekülen (3) durch die zumindest eine Öffnung (9) des zumindest einen synthetischen Behälters (2),
**dadurch gekennzeichnet, dass** das System zum Ausführen einer Neurotransmitterstimulierung von Neuronen des Weiteren ein Rückmeldungssystem umfasst, um eine Rückmeldung von den Neuronen (12) an das betätigte System für die chemische Stimulierung (10, 20) von Neuronen bereitzustellen; und dadurch, dass das Betätigungsmittel durch das Rückmeldungssystem gesteuert wird.

2. System nach Anspruch 1, wobei das Rückmeldungssystem zumindest einen Sensor umfasst.

3. System nach Anspruch 1 oder 2, wobei das Betätigungsmittel ein elektrisch betriebenes Betätigungsmittel ist.

4. System nach Anspruch 3, wobei das Betätigungsmittel zumindest eine erste Elektrode (5) und eine zweite Elektrode (6) umfasst.

5. System nach Anspruch 4, wobei der zumindest eine synthetische Behälter (2) in einer Trägerschicht (1) gebildet wird, wobei die erste Elektrode (5) an einer Bodenfläche (4) der Trägerschicht (1) positioniert ist und die zweite Elektrode (6) an einer Deckfläche (7) der Trägerschicht (1) positioniert ist.

6. System nach Anspruch 1 oder 2, wobei das Betätigungsmittel ein auf Druck basierendes Betätigungsmittel ist.

7. System nach Anspruch 6, wobei das Betätigungsmittel eine Membran (24) umfasst.

8. System nach Anspruch 7, wobei die Membran (24) aus einer elektrisch betätigbaren Schicht (27) gebildet ist, die zwischen zwei Elektroden (25, 26) liegt.

9. System nach Anspruch 8, wobei die elektrisch betätigbare Schicht (27) eine piezoelektrische Schicht ist.

10. System nach einem der Ansprüche 6 bis 9, wobei das System eine erste Trägerschicht (21) und eine zweite Trägerschicht (22) umfasst, wobei das Betätigungsmittel zwischen der ersten Trägerschicht (21) und der zweiten Trägerschicht (22) positioniert ist.

11. System nach einem der vorherigen Ansprüche, wobei das System eine Gruppe von synthetischen Behältern (2) umfasst, wobei jeder synthetische Behälter (2) eine Öffnung (9) hat.

12. System nach einem der Ansprüche 1 bis 11, wobei das System zumindest einen synthetischen Behälter (2) mit mehr als einer Öffnung (9) umfasst.

13. Verfahren zur Herstellung eines Systems zum Ausführen einer Neurotransmitterstimulierung von Neuronen (12), wobei das Verfahren umfasst:
- Bereitstellen eines betätigten Systems für die chemische Stimulierung von Neuronen (12) umfassend:
- Bereitstellen zumindest eines synthetischen Behälters (2) zum Speichern von Neurotransmittermolekülen (3),
- Bereitstellen zumindest eines synthetischen Behälters (2) mit zumindest einer Öffnung (9) und
- Bereit stellen eines Betätigungsmittels zum Steuern einer Freisetzung von Neurotransmittermolekülen (3) durch die zumindest eine Öffnung (9) des zumindest einen synthetischen Behälters (2); und
- Bereitstellen eines Rückmeldungsmittels zum Bereitstellen einer Rückmeldung von den Neuronen (12) an das betätigte System für die chemische Stimulierung (10, 20) von Neuronen, wobei das Rückmeldungsmittel zum Steuern des Betätigungsmittels dient.

14. Verfahren nach Anspruch 13, wobei das Bereitstellen eines Rückmeldungsmittels ein Bereitstellen zumindest eines Sensors umfasst.

15. Verfahren nach Anspruch 13 oder 14, wobei das Bereitstellen zumindest eines synthetischen Behälters (2) ein Ätzen einer Trägerschicht (1, 21) von einer ersten Oberfläche (11) in Richtung einer zweiten Oberfläche (12) der Trägerschicht (1, 21) umfasst.

16. Verfahren nach einem der Ansprüche 13 bis 15, wobei das Bereitstellen eines Betätigungsmittels durch Bereitstellen eines elektrisch betriebenen Betätigungsmittels ausgeführt wird.

17. Verfahren nach Anspruch 16, wobei das Bereitstellen eines Betätigungsmittels durch Bereitstellen einer ersten Elektrode (5) auf einer ersten Oberfläche (4) der Trägerschicht (1) und Bereitstellen einer zweiten Elektrode (6) auf einer zweiten Oberfläche (7) der Trägerschicht (1) ausgeführt wird.

18. Verfahren nach einem der Ansprüche 13 bis 15, wobei das Bereitstellen eines Betätigungsmittels durch Bereitstellen eines auf Druck basierenden Betätigungsmittels ausgeführt wird.

19. Verfahren nach Anspruch 18, wobei das Bereitstellen eines Betätigungsmittels durch Bereitstellen einer elektrisch betätigbaren Membran (24) ausgeführt wird.

20. Verfahren nach Anspruch 19, wobei das Bereitstellen einer elektrisch betätigbaren Membran (24) durch Bereitstellen eines Films (27), der zwischen zwei Elektroden (25, 26) liegt, ausgeführt wird.

21. Verfahren nach Anspruch 20, wobei das Bereitstellen eines Films (27) durch Bereitstellen eines piezoelektrischen Films ausgeführt wird.

## Revendications

1. Un système pour réaliser une stimulation de neurones (12) par des neurotransmetteurs, le système comprenant un Système Actionné pour la Stimulation Chimique (10, 20) de neurones (12),
le système actionné pour la stimulation chimique (10, 20) de neurones (12) comprenant :
- au moins un réservoir synthétique (2) avec au moins une ouverture (9), le réservoir (2) comprenant des molécules de neurotransmetteurs (3), et
- des moyens d'actionnement pour commander une libération de molécules de neurotransmetteurs (3) à travers l'au moins une ouverture (9) de l'au moins un réservoir synthétique (2),
**caractérisé en ce que** ledit système pour réaliser une stimulation de neurotransmetteurs de neurones comprend en outre un système de contre-réaction pour fournir une contre-réaction des neurones (12) au système actionné pour la stimulation chimique (10, 20) de neurones ; et **en ce que** les moyens d'actionnement sont commandés par ledit système de contre-réaction.

2. Un système selon la revendication 1, dans lequel le système de contre-réaction comprend au moins un capteur.

3. Un système selon la revendication 1 ou 2, dans lequel les moyens d'actionnement sont des moyens d'actionnement entraînés électriquement.

4. Un système selon la revendication 3, dans lequel les moyens d'actionnement comprennent au moins une première électrode (5) et une seconde électrode (6).

5. Un système selon la revendication 4, l'au moins un réservoir synthétique (2) étant formé dans un substrat (1), dans lequel la première électrode (5) est positionnée sur une surface inférieure (4) du substrat (1) et la seconde électrode (6) est positionnée sur une surface supérieure (7) du substrat (1).

6. Un système selon la revendication 1 ou 2, dans lequel les moyens d'actionnement sont des moyens d'actionnement basés sur la pression.

7. Un système selon la revendication 6, dans lequel les moyens d'actionnement comprennent une membrane (24).

8. Un système selon la revendication 7, dans lequel la membrane (24) est formée d'une couche actionnable électriquement (27) enserrée entre deux électrodes (25, 26).

9. Un système selon la revendication 8, dans lequel la couche actionnable électriquement (27) est une couche piézoélectrique.

10. Un système selon l'une quelconque des revendications 6 à 9, le système comprenant un premier substrat (21) et un second substrat (22), dans lequel les moyens d'actionnement sont positionnés entre le premier substrat (21) et le second substrat (22).

11. Un système selon l'une quelconque des revendications précédentes, dans lequel le système comprend un réseau de réservoirs synthétiques (2), chaque réservoir synthétique (2) ayant une ouverture (9).

12. Un système selon l'une quelconque des revendications 1 à 11, dans lequel le système comprend au moins un réservoir synthétique (2) ayant plus d'une ouverture (9).

13. Une méthode pour la fabrication d'un système pour réaliser une stimulation de neurones (12) par des neurotransmetteurs, la méthode comprenant de:
- fournir au moins un réservoir synthétique (2) pour contenir des molécules de transmetteurs (3),
- fournir ledit au moins réservoir synthétique (2) avec au moins une ouverture (9), et
- fournir des moyens d'actionnement pour commander une libération de molécules de neurotransmetteurs (3) à travers ladite au moins une ouverture (9) dudit au moins un réservoir synthétique (2); et
- fournir des moyens de contre-réaction pour fournir une contre-réaction des neurones (12) au système actionné pour la stimulation chimique (10, 20) de neurone, les moyens de contre-réaction étant pour commander les moyens d'actionnement.

14. Une méthode selon la revendication 13, dans laquelle fournir des moyens de contre-réaction comprend de fournir au moins un capteur.

15. Une méthode selon la revendication 13 ou 14, dans laquelle fournir au moins un réservoir synthétique (2) comprend de graver un substrat (1, 21) depuis une première surface (11) vers une seconde surface (12) du substrat (1,21).

16. Une méthode selon l'une quelconque des revendications 13 à 15, dans laquelle la fourniture des moyens d'actionnement est réalisée en fournissant des moyens d'actionnement entraînés électriquement.

17. Une méthode selon la revendication 16, dans laquelle la fourniture des moyens d'actionnement est réalisée en fournissant une première électrode (5) sur une première surface (4) du substrat (1) et en fournissant une seconde électrode (6) sur une seconde surface (7) du substrat (1).

18. Une méthode selon l'une quelconque des revendications 13 à 15, dans laquelle la fourniture des moyens d'actionnement est réalisée en fournissant des moyens d'actionnement basés sur la pression.

19. Une méthode selon la revendication 18, dans laquelle la fourniture des moyens d'actionnement est réalisée en fournissant une membrane actionnable électriquement (24).

20. Une méthode selon la revendication 19, dans laquelle la fourniture d'une membrane actionnable électriquement est réalisée en fournissant un film (27) enserré entre deux électrodes (25, 26).

21. Une méthode selon la revendication 20, dans laquelle la fourniture d'un film (27) est réalisée en fournissant un film piézoélectrique.
